# EUROPEAN PATENT APPLICATION

(11) **EP 0 838 228 A2**
(43) Date of publication of application: **29.04.1998**
(21) Application number: 97117938.7
(22) Date of filing: 16.10.1997
(51) Int. Cl.: A61M 5/32

(54) **Apparatus for removing injection needle and for disposing the same**

(30) Priority: 25.10.1996 JP 299817/96
(71) Applicant: Kabushiki Kaisha Dental-Act, Yamato-shi, Kanagawa-ken (JP); Nakano, Hirochika, Yokosuka-shi, Kanagawa-ken (JP)
(72) Inventor: Nakano, Hitoshi, Yokohama-shi, Kanagawa-ken (JP); Nakano, Hirochika, Yokosuka-shi, Kanagawa-ken (JP)
(74) Representative: Weiss, Peter, Dr. rer. nat.

(57) **Abstract**

A reliable and economical apparatus for automatedly removing used needles having screws from syringes and for storing the same without medical accidents into a disposing container is provided. A rotatable hollow cylinder formed around a stopping position of an inserted syringe equipped with the needle supports a rotatable disk around the cylinder. Arms having supported ends rotatably supported around supporting pins aligned along a coaxial circle figured on the disk and having free ends formed of serrated edges to face a needle holder are individually intruded through holes formed on the cylinder into inside. The disk driven by a motor traverses the free ends inwardly inside the cylinder to grip the holder using side edges of the holes as flucra of levers because the cylinder is in a suppressed status for rotation with a braking load. A further rotation of the motor drives the disk rotating the needle together with the cylinder in an unscrewing direction, overcoming a braking load-induced friction. During unscrewing, gripping forces of the ends are maintained by a dynamic friction-induced braked rotation of the cylinder. Removed needles are dropped by a reverse rotation of the motor on a cone to be accumulated in an equal thickness to reduce contaminant generations. An entrance of the container is enclosed with a lid during disposal, which has been tentatively fixed on outer walls of the container, when the container is full of needles.

## Description

### BACKGROUND OF THE INVENTION

### 1. Field of the Invention

The present invention relates mainly to an apparatus for disposing medicines in use for dental surgery wherein a used injection needle having a screw is removed from a syringe and, more particularly, to the apparatus both for removing the needle and for disposing the same which can maintain polluted needles in a safety status till incineration by collecting the needles into a container utilizing an automated mechanism for removing the needles from the syringe.

### 2. Brief Description of the Prior Art

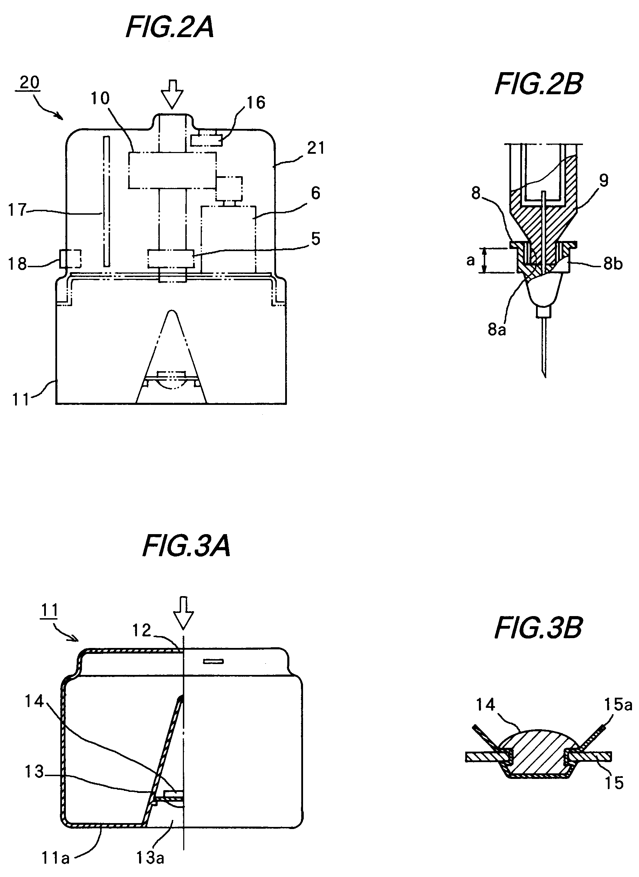

Up to now, used injection needles have been encapsulated with hoods and then removed from syringes together with the encapsulated hoods to be collected into bags or containers for disposal, which have uncertain shapes or are available at random. Because an injection pressure in the syringe for dental surgery use is particularly high, the injection needle in use for dental surgery has been loaded on the syringe especially by a use of a screw-mounting technology, wherein the injection needle 8 is to be mounted on the syringe 9, female screw ridges 8a of the needle 8 being threaded to mesh the syringe 9 as shown in FIG.2B. A manual operation of removing the injection needle by grasping an outer surface 8b of the threaded portion 8a of the used needle 8 shown in FIG.2B has frequently induced accidents such as injuries on finger tips, which are thought inherent to hurrying-up and to being tired-out of dental surgeons or dental hygienists during operations, even if sufficient cautions have been paid. In recent years, those accidents have been feared to death because those accidents invite dangers of being contagionous with HIV (so called AIDS) and hepatitis.

To avoid those dangers, a Japanese Laid-open Patent Publication No. 5-253300 (1993) has been proposed, wherein an automated remover formed of two rotating disks aligned parallel grasps the surface of the needle holder 8b of the injection needle 8 between those disks to remove the needle 8 from the syringe 9 while a Japanese Laid-open Utility Model Publication No. 6-80428 (1994) has been applied, wherein a disposing operation is finished by extruding only a needle tip portion from the so called injection needle to be disposed without unscrewing the threaded portion.

In the proposed removers mentioned above, the needles removed from the syringe to be disposed have been dropped mechanically into a lidless container for storage, which has been equipped with the apparatus. Those needles are forecastable thereafter to be restored into another means, which are unwritten on the specifications mentioned above, to be transferred and disposed. Taking a sanitation during the intermediate handling mentioned above into a consideration, a hygienic technology has been proposed as still another Japanese Laid-open Patent Publication No. 7-178136 (1995), wherein the needles to be disposed are automatedly stored into a tank formed of polymerized polyethylene resins almost in an enclosed status which operates correlatively with the apparatus for removing the injection needles.

However, in those removers mentioned before, both a mechanism of grasping the needle holder 8b including the screw portion 8a to remove the needle 8 and a mechanism of rotating the needle holder 8b to unscrew the threaded portion 8a have been formed so complex that the apparatus turns comparatively heavier. Accordingly, the inventors of the present invention have proposed another apparatus as further still another Japanese Patent Application No. 8-78070 (1996), of which removing mechanism is simpler than those that the preceding technologies mentioned before have exhibited and is further combined with an enclosed container for disposal.

On the other hand, in the hygienic tank attached to the remover of afore-mentioned preceding technology in use for disposing treatment, the removed needles are apt to pile up to form a shape of a mountain because the removed needles come through only one automated incoming entrance. When the tank full of the needles is moved for the disposing operation of the needles, the shape of the mountain is easily destroyed to generate dusts full of contaminants, which induces an anxiety about polluting an inside of a room for the dental surgery. This phenomenon has been pointed out as a big problem from a dental hygienic point of view.

### SUMMARY OF THE INVENTION

An object of the invention is to further simplify a mechanism of removing needles in the apparatus invented in the prior application just mentioned above, which has been proposed by the present inventors, by correlating both gripping operation of the needle holder and unscrewing operations of the thread more closely with each other, thereby increasing certainty in both operations to eliminate failings in gripping and unscrewing operations, which almost all of the preceding technologies mentioned before have frequently encountered with, and, thereby reducing expensive equipment costs to overcome a difficulty in marketing, which have been experienced being fabricated by the preceding technologies mentioned before due to their mechanical complexities.

Another object of the invention is to provide an apparatus combined with a container in use for storage and for disposal, in inner locations of which removed needles are reservable in an almost equal thickness, and, on an entrance of which a lid tentatively fixed on the container with a tentative fixability can be easily and quickly encapsulated to close the container when the container is separated from the apparatus immediately after an appropriate quantity of the removed needles are accumulated, thereby constituting the container separated from the apparatus to be a final container for disposal to suppress environmental pollutions.

A still another object of the present invention is to provide a reliable and unexpensive apparatus for removing the needle and disposing the same, wherein all process steps can be dealt with continuously from a step for removing the needle to a step for disposing the same outside without touching the needles, by integrating newly invented technologies mentioned above, thereby enabling dental surgery sites to eliminate both medical accidents and environmental pollutions.
(1) To attain the purposes mentioned above, the present invention is to provide an apparatus for removing an injection needle and for disposing the same, which is composed of a removing mechanism of a threaded needle from a syringe and of a container for storing and for disposing a removed needle; comprising:
   a rotatable hollow cylinder surrounding a stopping position of the needle mounted through a screw on the syringe;
   a rotatable annular disk supported through a bearing around an outer surface of the hollow cylinder;
   a means for rotating the disk such as an electric motor;
   a plurality of elongated arms each having a supported end, which is rotatably supported around a supporting pin aligned along an almost coaxial circle figured on a surface of the disk, and each having a free end formed of an almost serrated edge so as to face substantially a needle holder;
   a plurality of openings formed at a substantial height of the stopping position on the wall of the cylinder, through which corresponding free ends of the arms are individually intruded into an inside of the cylinder; and
   a braking load loaded on the cylinder; wherein:
   the motor is actuated so as to rotate the disk in response to insertion of the syringe mounted with the needle, resulting in rotation of the supported ends around the cylinder, thereby the arms being pressed against side edges of the openings to traverse the free ends toward a center of the cylinder for gripping the outer surface of the threaded portion with the serrated edges;
   following which the disk is further rotated by the motor, overcoming a braking resistance exerted by the braking load to loosen the meshed screw and to unscrew the needle with respect to the syringe, the cylinder also being rotated; and
   finally, the removed needle is dropped into the container for storage and disposal by an effect of gravity.
(2) To attain above-mentioned purposes more satisfactorily by suppressing generation of contaminated dusts, the present invention is to provide the apparatus according to (1), wherein:
   the container is provided with an inside bottom surface having a raised portion substantially in a middle thereof, which is formed in an almost conical form substantially opposing an incoming entrance for the removed needles.
(3) To attain above-mentioned purposes still more satisfactorily, the present invention is to provide the apparatus according to (1), wherein:
   the container includes a partition wall having an appropriate height for separating an inner space of the container into two substantially equal spaces each having a substantially equal volume, the partition wall being formed on an appropriate position of the bottom surface of the container including the conical form.
(4) To attain above-mentioned purposes further still more satisfactorily by a quick and easy closing of the container which is separated from the remover for diposal, the present invention is to provide the apparatus according to (1), wherein:
   a lid, which is to be capped on the entrance for enclosing the container during disposal, is tentatively fixed through a flexible sheet for pulling-off having an end thereof extending as a substantially tongue-end shaped-tab or a tentative fixing plate, on outside surfaces of the container such as on a front surface of the container or on a rear space of the conical form.

By constituting structural configurations as mentioned above, the apparatus for removing the needles and disposing the same according to the present invention traverses the plurality of the free ends of the arms driven by the rotation of the disk, wherein each side edge of holes opened on the cylinder surface serves as a fulcrum of a lever, facing the serrated edge toward the center of the cylinder, therewith to touch mechanically the outer surface of the needle holder. Those mechanisms maintain the certainty in a gripping operation of the arms because a gripping power utilizing a principle of a lever mechanism is so strong that the serrated edges according to the present invention does not fail to grip the needle, which almost all of the preceding proposals using such as a plain pressing mechanism of the needle holder with gear teeth have been unavoidable.

The further rotation of the disk thereinafter driven by the motor transmits through the arm a rotational torque of the motor toward a direction, wherein a meshed screw is loosened and unscrewed for demounting the needle, to the needle holder. During the further rotation of the disk driven by the motor, the cylinder continues to rotate, simultaneously being driven by the arms. However, the braking load loaded on the cylinder forbids substantially a free rotation of the cylinder and delays substantially its rotation so as to follow the rotation of the disk, which guarantees functions of the side edges of the holes serving as the flucra of the levers to grip tightly the needle holder during the rotation. Those functions work to maintain a stable grasping power and a strong unscrewing power of the arms throughout the rotation of the motor. The removed needles drops into the container for disposal and slides down on a surface of the conical form to be accumulated first on a corner of the container, and then climbing up the slope of the cone.

### BRIEF DESCRIPTION OF THE DRAWINGS

FIG.1A is a block diagram of a removing and disposing apparatus for a needle according to the present invention;
FIG.1B is a longitudinal sectional view of the apparatus of FIG.1A showing a mechanism of removing the injection needle;
FIG.1C is a cross-sectional view taken along line A-A in FIG.1B and enlarged for clarity;
FIG.1D is a schematic cross-sectional view showing a function of gripping the needle operated by this apparatus;
FIG.2A is a side view showing both an inner configuration and an outlook of an embodiment according to this invention;
FIG.2B is a fragmentary vertical sectional view of main components of the syringe, from which the injection needle is ready to be removed;
FIG.3A is a side view partly including a vertical section of a disposing container according to the present invention;
FIG.3B is a longitudinal section of a lid tentatively fixed to the container, to be capped later on an entrance for enclosing during disposal according to the present invention; and
FIG.4 is an operational diagram showing an operational sequence of the apparatus according to the present invention.

### DETAILED DESCRIPTION OF THE PREFERRED EMBODIMENTS

Hereinafter described is the best mode of carrying out the present invention into practice corresponding to the preferred embodiments.

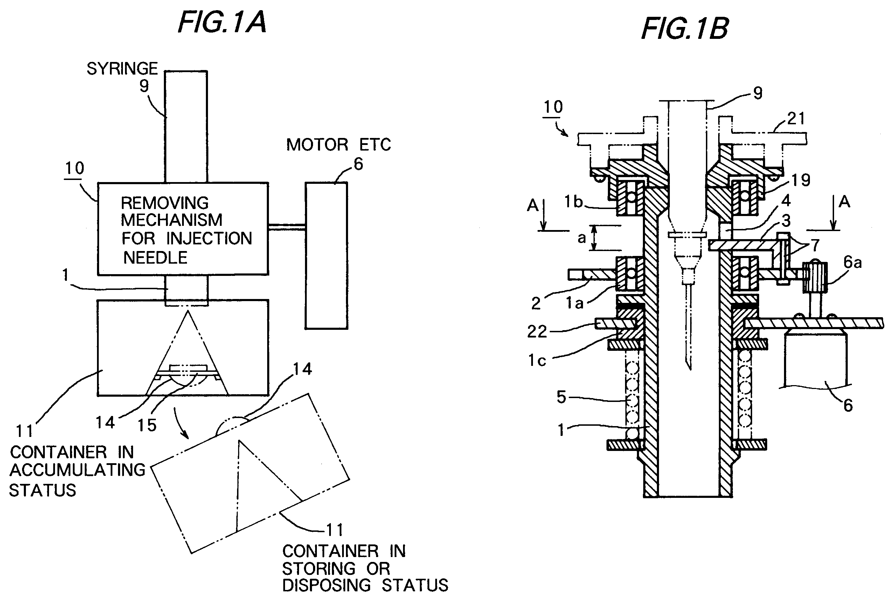

The preferred embodiments according to the present invention are to be illustrated in detail with reference to the drawings from FIG.1A to FIG.4.

As shown in FIGS.1A and 2A, the removing and disposing apparatus 20 for the needles according to the present invention is basically constituted of the removing mechanism 10 equipped with the motor 6 etc., into an upper entrance of which the syringe 9 is inserted during the removal of the mounted needle 8, and of the disposing container 11 for storing the removed needles 8, which drops downwards being guided by the cylinder 1. The container 11 includes the lid 14, which is tentatively fixed through a flexible sheet 15a having an end extending thereof a tongue end-shaped tab for pull-off or the fixing plate 15 for tentatively fixing on the bottom space 13a. When the container 11, which accumulates an adequate amount of the removed needles, is disposed, the lid 14 is taken off from the plate 15 to hermetically seal the container 11 by closing the incoming and outgoing entrance 12 of the needles.

As shown in FIG.1B, the removing mechanism 10 for removing the needles rotatably supports the cylinder 1 through an uppermost bearing 1b embedded in a concave portion formed on a rear surface of an insertion frame 19 for receiving the syringe 9 denoted by a hatching of slanting solid lines, which is secured by means of screws to the main case 21 of the remover 10 represented by doubly dotted lines on an uppermost of FIG.1B. On an outer space corresponding to a lowest edge of the stopping position "a" of the syringe 9, the cylinder 1 rotatably supports the disk 2 through the bearing 1a, which is driven by a gear 6a attached to the motor 6.

To an outer wall surface of a lower portion of the cylinder 1, the braking load 5 formed of, for instance, a compression spring is attached being wound. Namely, the cylinder 1 holds a lower end of the spring while a supporting plate 22 of the removing mechanism 10, in which the thrust bearing 1c for supporting the cylinder 1 is embedded, receives a pressure of the spring through a receiving plate connected with an upper end of the spring. Instead of the supporting plate 22, the main case 21 of the remover 10 or a rear surface of the disk 2 can herein receive the spring pressure while another brake can be attached to the remover 10 or the disk 2 to apply another pressure to the cylinder 1 on the contrary.

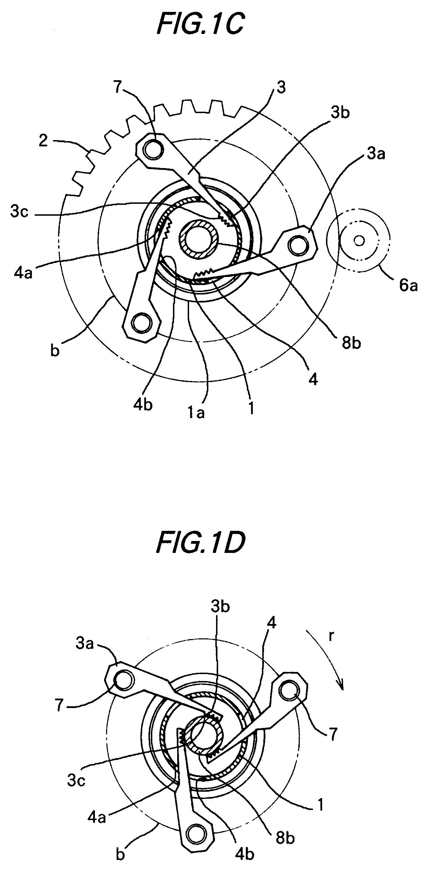

As further shown in FIG. 1C, the arms 3 are rotatably supported with their supported ends 3a around the supporting pins 7, which are aligned so as to form an equiangular polygon along the circle b drawn coaxially on the disk 2. The oblong-shaped holes 4, of which number and positioning correspond to those of the arms 3, are formed on the wall surface of the cylinder 1 at a height level of the stopping position "a" so that horizontal lengths of the holes 4 become longer than vertical lengths of the holes 4. Shapes of the holes 4 depend somewhat on cross-sectional shapes of the arm 3 so that differently shaped holes can be adoptable when arms having particularly shaped cross-sections are used, for instance, to raise a rigidity or to reduce a weight so long as the holes 4 are aligned so as to form an almost equiangular polygon on a cross-sectional plane of the cylinder 1. The free ends 3b of arms 3 are individually intruded through the corresponding holes 4 while the serrated edges 3c are formed on the free ends 3b so that the serrated edges 3c orient internally. Herein the serrated edges 3c are replaceable with surfaces having other shapes including a zigzag-formed surface so long as they can grasp tightly the needle holder 8b.

As can be seen from FIG.3A, a corner shape and a plurality of small bumps, which enable the container 11 to connect itself with the main case 21 of the removing mechanism 10, are provided on an upper side wall of the container 11 for disposing the removed needles. Simultaneously, the incoming entrance 12, of which inner diameter can accept an insertion of a lower end of the hollow cylinder 1 from a direction indicated by a broad arrow shown in FIG.3A when the apparatus 20 is assembled, is provided on an upper surface of the container 11. On the bottom surface 11a of the container 11, which faces the entrance 12, the conical form 13 such as a circular cone or a pyramid is formed inside the container 11. Incidentally, a partition wall having an appropriate height from the conical form 13, which separates an inner space substantially equally into a left and a right hand sides, can be supplied on an arbitrary position to prevent a movement of the stored needles to be disposed later.

As shown further in FIG.3B, the lid 14 is formed so as to be fitted into the incoming and outgoing entrance hole 12. Namely, an upper part of the lid 14 is formed so as to have an appropriately larger diameter than that of the entrance 12, of which longitudinal cross-section is substantially a circular arc. A circular groove is formed just below the arc so as to be capable of fitting in the entrance 12. A lower projection having a slightly larger diameter than that of the groove is formed below the groove to enable to close the container 11 when the lid is fixed into the entrance 12. The tentative fixing plate 15 stacked with a flexible sheet 15a formed of such as a soft plastic resin polymer having an end extending thereof a tongue-end shaped-tab is herein attached to the lid 14. An opening, which can fit to the groove of the lid 14, is formed on a central portion of the tentative fixing plate 15. External shape and size of the fixing plate 15 are formed so that the plate 15 can be temporarily fixed by small bumps formed on a rear surface of the conical form 13 when it is pushed into the rear space 13a.

More specifically described, a microswitch 16 operated being driven by a sensor as shown in FIG.2A, which detects an insertion of the syringe 9 mounted with the needle 8 and is provided in the vicinity of the entrance of the syringe inserted from a direction indicated by the broad arrow shown in the figure, is provided inside the main case 21 of the remover 10 to start a rotation of the electric motor 6 and to operate a removing mechanism 10. Power supply for driving the motor 6 is applied from electric terminals 18 for connecting power supply lines while operational controls of the motor 6 are managed by output signals produced from a control circuit board 17. A snapswitch for switching the power supply circuit between a closed state and an open state can be provided externally near at hand for convenience, a power input socket being connected with the terminal 18, though the switch is unshown in the figures. The power supply is also applicable from a built-in battery equipped in the main case 21.

The braking load 5, which is herein fixed to the cylinder 1 and gives a pressure on the plate 22 to suppress the free rotation of the cylinder 1 during operation of the remover 10, is replaceable with a plumb bob having an appropriate weight instead of afore-mentioned pressure induced by the coil spring. The power necessary for gripping the needle holder 8b with the serrated edges 3c before loosening the meshed screw 8a requires of the cylinder 1 a comparatively larger resistive force with respect to rotation, which can be afforded by a static friction coefficient of the braking load 5. Meanwhile the power necessary for gripping the needle holder 8b with the serrated edges 3c during unscrewing rotation of the needle 8 together with the cylinder 1 requires of the cylinder 1 a less resistive force compared with that prior to loosening the meshed screw 8a, which can be afforded by a dynamic friction coefficient of the braking load 5.

The container 11 for disposing the needles is herein a disposable-type container, which is to be closed with the lid 14 and incinerated after accepting an appropriate volume of the removed needles. No lid can be pulled off again by bare hands anymore so long as the lid 14 is fitted directly without inserting the tentative fixing plate 15 or the flexible sheet 15a. If a recycle-type container is used, the fixing plate 15 or the flexible sheet 15a is usable between the lid and the entrance 12. Then an empty container is newly assembled with the main case 21 of the remover 10.

The positioning, on which the lid 14 is tentatively fixed through the tentative fixing plate 15 or the flexible sheet 15a having the end extending thereof the tongue-end shaped-tab for pulling-off formed of such as a soft plastic resin film, is not necessarily limited to the vacant space 13a for space saving on the rear surface of the conical form 13. Those components can be tentatively fixed even on a front surface of the container 11 if afore-mentioned corner shape and small bumps for connecting the container 11 with the main case 21 of the remover 10 are well designed and formed to provide an enough vacant space between the remover 10 and the container 11. Removing the lid 14 tentatively attached to the front surface enables a faster closing and a less vibration of the container during encapsulation, compared with that tentatively attached to the rear space 13a.

Materials in use for the components constituting the apparatus 20 of the present invention, particularly in use for the main case 21 of the remover 10 and the disposable-type container 11 to be incinerated, are preferably to be plastic resins selected from synthetic plastic resins including ABS (acrylonitrile-butadien-stylene) co-polymerized resin, polypropylene resin and polyethylene resin. They are also preferably to be materials selected from wooden materials and light metals, of which surfaces have been subjected to an appropriate surface treatment. Materials in use for the hollow cylinder 1 is preferably to be stainless steels because contacts of the used needles frequently take place.

The electric motor 6 is preferably a motor in use for DC (direct current) 12 V (volts) and, for instance in use for removing the needles having diameters no greater than 8 mm (millimeters), has preferably an output power about 5 W (watt).

When the annular disk 2 is rotated by the motor 6 at an appropriate rotational speed, a motor equipped with a deceleration mechanism is usable meanwhile a mechanical system which intervenes reduction gears inbetween is also available. The control of the rotational speed of the motor is also attainable either by an electronic method, wherein a frequency of the power supply is converted, or by an electric method, wherein a voltage of the power supply is varied.

A simplest way to start and stop the rotation of the motor 6 is obtainable by programming elapsing times on a time switch. The power supply for the motor also can be turned off when an operational load turns insensible after an elapse of a certain period of time. On the contrary, when the load accompanying with the rotation stays still sensible after an elapse of t seconds, the same time schedule can be repeatedly programmed again on the time switch. Furthermore, a supply power control of the motor 6 can be done by sensing a number of revolutions of the motor because if an unscrewing operation is assuredly performed being adjusted to a number of threaded ridges, the threaded portion 8a of the needle 8 can be just removed from the syringe 9. The control of rotating the motor is also attainable by use of a sensor, which can detect a falling down status of the removed needle. Herein the motor can be stopped with a few reverse rotations for a time when the load during the operation turns assuredly insensible.

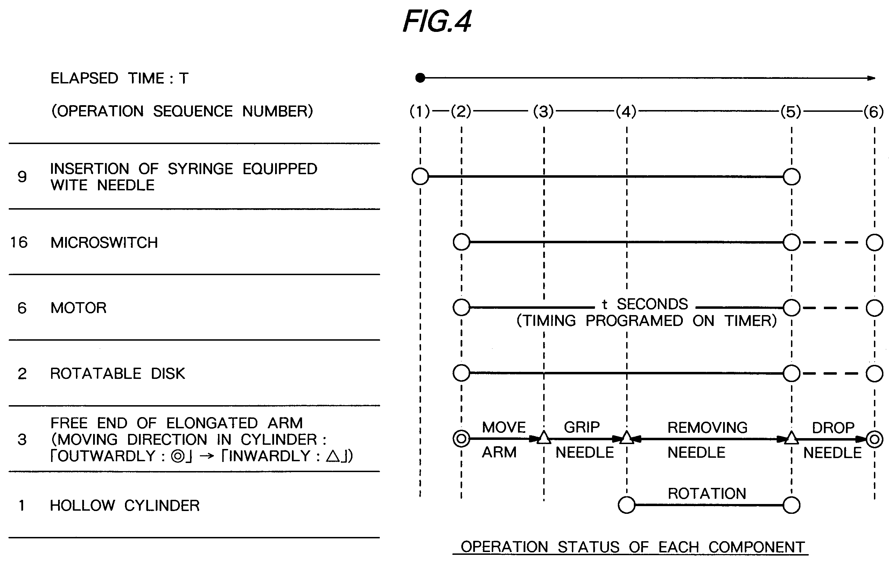

The removing mechanism 10 in the apparatus 20 of the present embodiment constituted as mentioned above works its functions just as an operational sequence shown in FIG.4.

When the syringe 9 equipped with the needle 8 is manually inserted from the upper entrance of the main case 21 at Seq (Sequence) (1), the sensor detects the insertion of the syringe 9 and turns the microswitch 16 on to introduce the electric power at Seq (2). The circuitry is interconnected so that the motor 6 continues rotation for t seconds, which has been programmed on the timer, if the motor 6 starts to rotate.

The output power of the motor 6 then rotates through the gear 6a the rotating disk 2 in a clockwise direction denoted by a curved arrow r shown in FIG.1D. This begins to drive the arms 3 for traversing at Seq (2), of which supported ends 3a are rotatably supported on the disk 2 around the supporting pin 7. The free ends 3b of the arms 3, which at first are located adjacently to an inside wall of the cylinder 1, are moved inwardly until the serrated edges 3c touch the outside surface 8b of the threaded portion 8a at Seq (3), utilizing the side edges 4a of the holes 4 as the flucra of the levers. Herein during a period between Seqs (3) and (4), the bearing 1a works further to fasten the grip of the serrated edge 3c, which grasps the outer surface 8b of the screw portion 8a.

At Seq (4), the arms 3 begin rotating the needle 8 by overcoming a resistive force formed of the braking load 5, which is attached to the cylinder 1 and presses the supporting plate 22 of the remover 10, simultaneously rotating the cylinder 1. A delay in rotation of the cylinder 1 induced by the braking load 5 maintains the gripping force of the serrated edges 3c during unscrewing the needle 8.

At Seq (5), simultaneously when the unscrewing rotation of the arms 3 is finished, a turning the microswitch 16 off and a stopping the rotation of the rotatable disk 2 driven by the motor 6 take place correlatively because a timing "t" is preliminarily programmed on the timer. Taking out the syringe 9 manually from the remover 10 being informed by a lighting of a lamp or warned by a buzzer at Seq (5) completes a separation of the needle 8 from the syringe 9.

A rotation of the motor 6 at least at 360 degrees in an opposite direction represented by broken lines shown in FIG.4 moves the free ends 3b outwardly toward the inside wall of the cylinder 1 till Seq (6) by utilizing another side edges 4b of the openings 4 as flucra of levers. Between Seqs (5) and (6), the removed needle falls down from the free ends 3b of arms 3 into the container 11 for storing and disposing.

Although some of fully automated handlings are replaced with manual ones in the preferred embodiments mentioned above such as the insertion, holding or the taking out of the syringe, that does not reduce any safety in handling the dental medicals but rather serves to reduce the equipment cost so long as handlings of the needles are at least automatedly carried out. It is no need to say that a fully automation including the handling of the syringe is adoptable, for instance, to raise a through-put in removing operation.

The removing mechanism of the apparatus according to the present invention is further simplified than those up to now and guarantees a stable gripping force and a strong unscrewing power of the needle only by a unidirectional rotation of the motor, employing the plurality of the elongated arms and the openings having an appropriate shape formed on the cylinder surface for guiding the arms.

The disposing container of the apparatus according to the present invention is the cartridge-type disposable container, which can store the wasted needles at a substantially uniformly distributed status on the bottom and be incinerated at an enclosed status with the lid.

Accordingly, the apparatus for removing the needles and the disposing the same according to the present invention, which is essentially constituted in combination of the removing mechanism mentioned above and of the container similarly mentioned above, has another advantages in marketability such as an easy handling and an economical equipment price beside advantages in safety and in environmental hygiene such as an isolability of the threaded needles, which have been thought difficult to be removed, from finger tips throughout all handling process steps from the automated removal to the disposing treatment just prior to the incineration and a substantial elimination of polluting the environment.

Consequently, the apparatus according to the present invention not only can reduce dangers for inducing probability of the medical accidents but also can give large technological and managerial impacts concerning the industrial hygienics upon an environmental improvement of medical care sites.

## Claims

1. An apparatus (20) for removing an injection needle (8) and for disposing the same, which is composed of a removing mechanism (10) of a threaded needle (8) from a syringe (9) and of a container (11) for storing and for disposing a removed needle (8); comprising:
a rotatable hollow cylinder (1) surrounding a stopping position (a) of said needle (8) mounted through a screw (8a) on said syringe (9);
a rotatable annular disk (2) supported through a bearing (1a) around an outer surface of said hollow cylinder (1);
a means (6) for rotating said disk (2) such as an electric motor (6);
a plurality of elongated arms (3) each having a supported end (3a), which is rotatably supported around a supporting pin (7) aligned along an almost coaxial circle (b) figured on a surface of said disk (2), and each having a free end (3b) formed of an almost serrated edge (3c) so as to face substantially a needle holder (8b);
a plurality of openings (4) formed at a substantial height of said stopping position (a) on said wall of said cylinder (1), through which corresponding free ends (3b) of said arms (3) are individually intruded into an inside of said cylinder (1); and
a braking load (5) loaded on said cylinder (1); wherein:
said motor is actuated so as to rotate said disk in response to insertion of said syringe (9) mounted with said needle (8), resulting in rotation of said supported ends (3a) around said cylinder (1), thereby said arms (3) being pressed against side edges (4a, 4b) of said openings (4) to traverse said free ends (3b) toward a center of said cylinder (1) for gripping said outer surface (8a) of said threaded portion (8a) with said serrated edges (3c);
following which said disk (2) is further rotated by said motor (6), overcoming a braking resistance exerted by said braking load (5) to loosen said meshed screw (8a) and to unscrew said needle (8) with respect to said syringe (9), said cylinder (1) also being rotated; and
finally, said removed needle (8) is dropped into said container (11) for storage and disposal by an effect of gravity.

2. The apparatus (20) according to Claim 1, wherein:
said container (11) is provided with an inside bottom surface (11a) having a raised portion substantially in a middle thereof, which is formed in an almost conical form (13) substantially opposing an incoming entrance (12) for said removed needles (8).

3. The apparatus (20) according to Claim 1, wherein:
said container (11) includes a partition wall having an appropriate height for separating an inner space of said container (11) into two substantially equal spaces each having a substantially equal volume, said partition wall being formed on an appropriate position of said bottom surface (11a) of said container (11) including said conical form (13).

4. The apparatus (20) according to Claim 1, wherein:
a lid (14), which is tentatively fixed through an almost flexible sheet (150) for pulling-off having an end thereof extending as a substantially tongue-end shaped-tab or a tentative fixing plate (15) on outside surfaces of said container (11), is encapsulated without intervening said sheet (15a) or said plate (15) directly on an entrance (12) of a disposable-type container for enclosing said container (11) during disposal.

5. The apparatus (20) according to Claim 1, wherein:
a lid (14), which is tentatively fixed through an almost flexible sheet (15a) for pulling-off having an end thereof extending as a substantially tongue-end shaped-tab or a tentative fixing plate (15) on outside surfaces of said container (11), is encapsulated by intervening said sheet (15a) or said plate (15) indirectly on said entrance (12) of a recycle-type container for enclosing said container during disposal.
